# EUROPÄISCHE PATENTSCHRIFT

(11) **EP 0 814 737 B1**
(45) Veröffentlichungstag und Bekanntmachung des Hinweises auf die Patenterteilung: **14.07.1999**
(21) Anmeldenummer: 96904092.2
(22) Anmeldetag: 01.03.1996
(51) Int. Cl.: A61F 13/15

(54) **KLEIDUNGSSTÜCK, INSBESONDERE WEGWERF-KLEIDUNGSSTÜCK ZUM EINMALIGEN GEBRAUCH**
ITEM OF CLOTHING, ESPECIALLY DISPOSABLE CLOTHING FOR USE ONCE ONLY
VETEMENT, NOTAMMENT VETEMENT JETABLE A USAGE UNIQUE

(30) Priorität: 18.03.1995 DE 19509953
(43) Veröffentlichungstag der Anmeldung: 07.01.1998
(73) Patentinhaber: Paul Hartmann Aktiengesellschaft, 89522 Heidenheim (DE)
(72) Erfinder: MALOWANIEC, Krzysztof, D., D-89522 Heidenheim (DE)
(74) Vertreter: Dreiss, Fuhlendorf, Steimle & Becker, Patentanwälte
(86) Internationale Anmeldenummer: EP9600865
(87) Internationale Veröffentlichungsnummer: WO9629036

(56) Entgegenhaltungen:
- EP-A- 0 312 071
- WO-A-93/01781
- US-A- 4 036 233
- US-A- 4 731 066

## Beschreibung

Die vorliegende Erfindung betrifft ein Kleidungsstück, insbesondere ein Wegwerf-Kleidungsstück zum einmaligen Gebrauch, nach dem Oberbegriff des Anspruchs 1.

Bei derartigen Kleidungsstücken, insbesondere bei Wegwerf- oder Einmal-Windein für Kinder oder Inkontinente ist es erforderlich, daß zumindest im Bereich eines um die Hüften und den Bauch herum zu legenden Bundes elastische Bereiche vorgesehen sind, um einen sicheren und bequemen Sitz der Windel zu gewährleisten. Dieses trifft auch zu für elastische Abschnitte an den Beinen oder Ärmeln von Einwegbekleidung.

Bei bekannten Wegwerf-Windeln (WO 94/05241, WO 93/25171, WO 92/22274, WO 92/22273) ist im Bereich des Bundes nahe von Verschlußklebebändern je ein elastisches Element bzw. eine elastische Zone vorgesehen, die eine Lage aus einem elastischen Sto3ff enthält. Diese Lage ist sandwichartig zwischen einer flüssigkeitsundurchlässigen Folie und einem Vlies angeordnet und mit diesen verbunden.

Um zu erreichen, daß die unelastische, aber plastische Folie und das ebenfalls unelastische, aber plastische Vlies bei einer Dehnung der elastischen Zone der elastischen Lage folgen können, ohne dabei die Dehnung der elastischen Lage wesentlich zu beeinträchtigen, werden die Folie, die elastische Lage und das Vlies gemeinsam mechanisch gedehnt, wodurch die beiden unelastischen, plastischen Lagen im Bereich der elastischen Lage zwangsweise überdehnt und somit verlängert sowie mit der elastischen Lage verbunden werden.

Auf diese Weise soll eine elastische Zone geschaffen werden, in der die überdehnten, unelastischen Stoffe eine elastische Dehnung des elastischen Materials praktisch nicht mehr behindern können. Hierbei ist es Jedoch schwierig, die unelastischen Lagen, also die Folie und das Vlies, derart gezielt zu überdehnen, daß sie anschließend bei einer gewünschten Dehnung der elastischen Zone diesen Grad der Dehnung freigeben. Eine weitere Schwierigkeit besteht darin, bei der elastischen Lage die Elastizität trotz der Überdehnung beizubehalten bzw. den gewünschten Grad der Dehnbarkeit einzustellen.

Aus der US-PS 4,371,066 ist eine Windel bekannt, bei welcher im Taillenbereich auf beiden Seiten der Windel auf der Außenseite Schlitze vorgesehen sind, um die Elastizität der Windel in diesem Bereich zu erhöhen. Bei einer Dehnung des Materials bilden sich Öffnungen.

Davon ausgehend liegt der Erfindung die Aufgabe zugrunde, ein trotz der Einschnitte reißfestes Laminat zu erhalten, bei welchem die Einschnitte im Bereich des elastischen Elements sich nicht durch alle drei Lagen erstrecken und die Flüssigkeitsundurchlässigkeit in diesem Bereich sicherstellen.

Diese Aufgabe wird bei einem gattungsgemäßen Kleidungsstück erfindungsgemäß durch die kennzeichnenden Merkmale des Anspruches 1 gelöst.

Durch das erfindungsgemäße Einbringen der Einschnitte läßt es sich sicher vermeiden, daß sich die Einschnitte in unerwünschter Weise durch alle drei Lagen hindurch erstrecken oder die unelastische Lage so schädigen, daß diese bei einer erwünschten Dehnung reißt. Damit wird die gewünschte Flüssigkeitsundurchlässigkeit des Kleidungsstückes auch im Bereich des elastischen Elements sichergestellt.

Bei der Anordnung der Einschnitte in Reihen lassen sich die Einschnitte in den unelastischen Lagen bei einer Dehnung zu rautenförmigen Öffnungen aufziehen, und die entsprechenden Lagen können bei einer Dehnung der elastischen Lagen in der gewünschten Weise folgen. Durch eine entsprechende Wahl der Länge der Einschnitte, der Abstände der Einschnitte in einer Reihe und der Abstände der Reihen untereinander läßt sich gegebenenfalls auch die Elastizität des elastischen Elements beeinflussen.

Die Erfindung wird im folgenden beispielsweise anhand der Zeichnung näher erläutert. In dieser zeigen:
- Figur 1:: einen schematischen Schnitt durch ein elastisches Element eines erfindungsgemäßen Kleidungsstücks;
- Figur 2:: eine Draufsicht auf einen Ausschnitt eines erfindungsgemäßen Kieidungsstücks im Bereich eines elastischen Elements;
- Figur 3:: eine Draufsicht entsprechend Figur 2 auf ein elastisches Element im gedehnten Zustand;
- Figur 4:: eine schematische Darstellung des Einschnittbereiches.

In den verschiedenen Figuren der Zeichnung sind einander entsprechende Teile mit gleichen Bezugszeichen versehen.

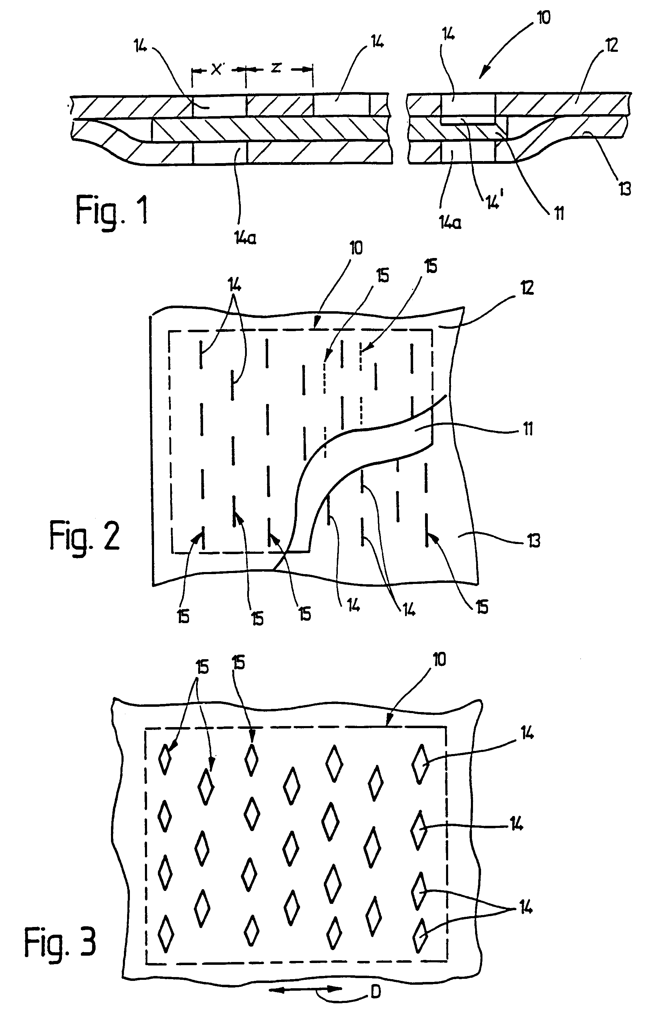

Das erfindungsgemäße Kleidungsstück weist, wie Figur 1 zeigt, im Bereich eines elastischen Elementes 10 eine Lage 11 aus einem elastischen Stoff auf, die sandwichartig zwischen zwei unelastischen Lagen 12, 13 angeordnet und mit diesen verbunden ist.

Die eine unelastische Lage ist dabei z.B. eine flüssigkeitsundurchlässige, weiche, flexible und plastische Folie 12, wie sie beispielsweise auf der dem Körper abgewandten Außenseite bei Wegwerf-Windeln vorgesehen ist. Die andere unelastische Lage ist z. B. von einem weichen, flexiblen und plastischen Vlies 13 gebildet, welches üblicherweise auf der Innenseite von Wegwerf-Windeln vorgesehen ist. Wenn wenigstens eine der beiden unelastischen Lagen 12, 13 aus einem flüssigkeitsundurchlässigen Material besteht, wird auch für die elastische Lage ein flüssigkeitsundurchlässiges Material gewählt.

In der Folie 12 und im Vlies 13 sind im Bereich der elastischen Lage 11 Einschnitte 14 vorgesehen, die sich vorzugsweise nur durch die Folie 12 und das Vlies 13 (s. Figur 2) erstrecken und die, wie in Figur 1 auf der linken Seite dargestellt, nicht in die elastische Lage 11 eindringen.

Wie in Figur 1 auf der rechten Seite dargestellt, können sich jedoch die Einschnitte 14 auch bis in die elastische Lage 11 hinein erstrecken, wobei sich der Einschnitt 14' in der elastischen Lage 11 jedoch nicht durch die elastische Lage 11 hindurch erstreckt, sondern nur geringfügig in diese eindringt.

Die Einschnitte können in deckungsgleicher Anordnung sowohl in der unelastischen Lage 12 als auch in der Lage 13 (bei 14a) vorgenommen werden.

Wie Figur 2 zeigt, sind die Einschnitte 14 in Reihen 15 angeordnet, wobei die Einschnitte 14 in benachbarten Reihen in Reihenlängsrichtung gegeneinander versetzt angeordnet sind. Außerdem sind die Reihen 15 der Einschnitte 14 in der Folie 12 gegenüber den Reihen 15 der Einschnitte 14 im Vlies 13 ebenfalls versetzt angeordnet. Hierdurch wird sichergestellt, daß, falls die elastische Lage 11 Einschnitte 14' aufweist, diese so angeordnet sind, daß die elastische Lage nicht übermäßig beschädigt wird und dennoch funktionsfähig bleibt.

Wie Figur 3 zeigt, weiten sich die Einschnitte 14 bei einer Dehnung des elastischen Elements 10 in Richtung des Doppelpfeils D zu rautenförmigen Öffnungen.

Wird ein derartiges elastisches Element bei einer Windel oder dergleichen eingesetzt, so wird durch die Einschnitte 14 auch im gedehnten Zustand des elastischen Elements 10 die Flüssigkeitsundurchlässigkeit des im Bereich des elastischen Elements 10 dreilagigen Windelstoffs nicht beeinträchtigt, da in diesem Bereich die sich aufgrund der Einschnitte 14 bildenden rautenförmigen Öffnungen durch die elastische Lage abgedeckt sind, so daß durch die Öffnungen keine Flüssigkeit austreten kann.

Es kann auch gemäß einer zweckmäßigen Ausführungsform vorgesehen sein, daß die elastische Lage aus saugfähigem Material besteht oder dieses enthält, wobei das saugfähige Material aus Fasern und/oder aus hohe Flüssigkeitsabsorptionseigenschaften aufweisenden Hydrogelen hergestellt sein kann.

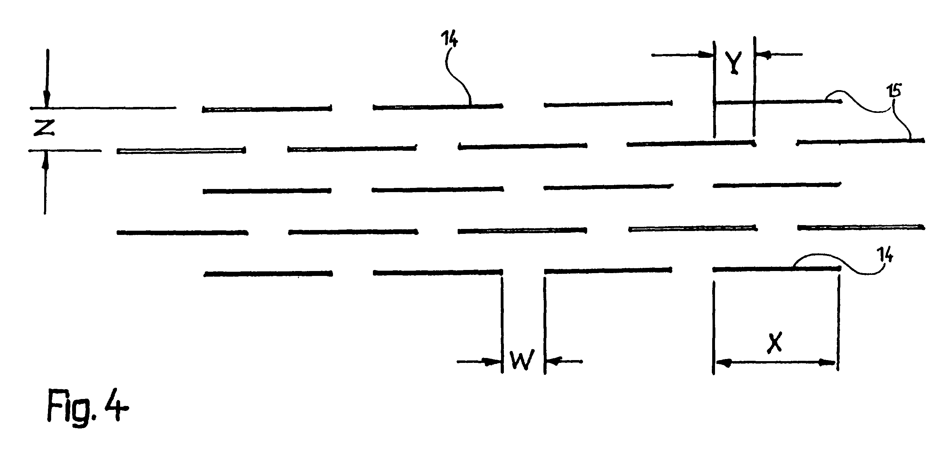

Optimale Abmessungen sind wie folgt: Bei einer Länge x des Einschnittes 14 (s. Figur 4) und einem Abstand w zwischen den Einschnitten sowie einem Abstand z der Einschnittsreihen 15 voneinander und einem Überlappungsbereich y der Einschnitte zweier benachbarter Reihen sollte der Wert z ungefähr den Werten y bzw. w entsprechen, wobei z zwischen 1 bis 4 mm liegt. Die Länge x soll etwa 3 z betragen. Die Elastizität wird umso besser, je größer die Werte y und x gewählt werden.

## Patentansprüche

1. Kleidungsstück, insbesondere Wegwerf-Kleidungsstück zum einmaligen Gebrauch,
mit wenigstens einer im wesentlichen unelastischen Lage (12) aus einem weichen, flexiblen und plastischen Stoff und mit einer aus einem elastischen Stoff bestehenden Lage (11), die zumindest einen Teilbereich zumindest einer der unelastischen Lagen (12) überdeckt und zur Bildung eines elastischen Elements (10) mit der unelastischen Lage verbunden ist, wobei in der unelastischen Lage (12) im Bereich des Überlappens mit der elastischen Lage (11) eine Mehrzahl von vorzugsweise in Reihen angeordneten Einschnitten (14) vorgesehen ist,
**dadurch gekennzeichnet**, **daß**
die Einschnitte (14) in der einen unelastischen Lage (12) gegen die Einschnitte (14) in der anderen unelastischen Lage (13) versetzt angeordnet sind.

2. Kleidungsstück nach Anspruch 1, dadurch gekennzeichnet, daß sich die Einschnitte (14) ganz oder nur teilweise bis in die elastische Lage (11) erstrecken.

3. Kleidungsstück nach einem der vorhergehenden Ansprüche, dadurch gekennzeichnet, daß die elastische Lage (11) saugfähiges Material enthält.

4. Kleidungsstück nach Anspruch , 1 oder 2, dadurch gekennzeichnet, daß der Abstand (z) zwischen den Einschnittsreihen (15) ungefähr der Länge (y) des Überlappungsbereichs der Einschnitte (14) zweier Einschnittsreihen (15) entspricht.

5. Kleidungsstück nach einem der vorhergehenden Ansprüche, dadurch gekennzeichnet, daß der Abstand (z) der Einschnittsreihen (15) voneinander etwa dem Abstand (w) zwischen den Einschnitten (14) einer Einschnittsreihe (15) entspricht.

6. Kleidungsstück nach einem der vorhergehenden Ansprüche, dadurch gekennzeichnet, daß der Abstand (z) zwischen den Einschnittsreihen (14) ca. 1-4 mm beträgt.

7. Kleidungsstück nach einem der vorhergehenden Ansprüche, dadurch gekennzeichnet, daß die Länge (x) eines Einschnitts (14) die 2- bis 4-fache Länge des Abstandes z beträgt.

8. Kleidungsstück nach einem der vorhergehenden Ansprüche, dadurch gekennzeichnet, daß die Länge (x) eines Einschnitts (14) etwa 3z beträgt.

## Claims

1. An item of clothing, in particular a disposable item of clothing for single use,
having at least one substantially inelastic layer (12) of a soft, flexible and plastic material and having a layer (11) which is made of an elastic material, covers at least part of at least one of the inelastic layers (12) and is connected to the inelastic layer to form an elastic element (10), a plurality of incisions (14), preferably arranged in rows, being provided in the inelastic layer (12) in the region in which the latter overlaps the elastic layer (11),
**characterized in that**
the incisions (14) in the one inelastic layer (12) are arranged offset with respect to the incisions (14) in the other inelastic layer (13).

2. An item of clothing according to Claim 1, characterized in that the incisions (14) extend entirely or only partially into the elastic layer (11).

3. An item of clothing according to one of the preceding claims, characterized in that the elastic layer (11) contains absorbent material.

4. An item of clothing according to Claim 1 or 2, characterized in that the spacing (z) between the rows (15) of incisions corresponds approximately to the length (y) of the region over which the incisions (14) of two rows (15) of incisions overlap.

5. An item of clothing according to one of the preceding claims, characterized in that the spacing (z) between the rows (15) of incisions corresponds approximately to the spacing (w) between the incisions (14) of a row (15) of incisions.

6. An item of clothing according to one of the preceding claims, characterized in that the spacing (z) between the rows (14) [sic] of incisions is approximately 1-4 mm.

7. An item of clothing according to one of the preceding claims, characterized in that the length (x) of an incision (14) is 2 to 4 times the length of the spacing z.

8. An item of clothing according to one of the preceding claims, characterized in that the length (x) of an incision (14) is approximately 3z.

## Revendications

1. Vêtement, notamment vêtement jetable à usage unique,
comportant au moins une couche pour l'essentiel non élastique (12) composée d'une matière plastique, flexible et douce, et comportant une couche (11) se composant d'une matière élastique, qui recouvre au moins une zone partielle d'au moins une des couches non élastiques (12) et qui, pour former un élément élastique (10), est reliée à la couche non élastique, une pluralité d'entailles (14) de préférence disposées en rangées étant ce faisant prévues dans la couche non élastique (12) dans la zone de recouvrement avec la couche élastique (11), caractérisé en ce que
les entailles (14) sont disposées de façon décalée dans l'une des couches non élastiques (12) par rapport aux entailles (14) dans l'autre couche non élastique (13).

2. Vêtement selon la revendication 1, caractérisé en ce que les entailles (14) s'étendent totalement ou seulement partiellement jusque dans la couche élastique (11).

3. Vêtement selon l'une quelconque des revendications précédentes, caractérisé en se que la couche élastique (11) renferme une matière absorbante.

4. Vêtement selon la revendication 1 ou 2, caractérisé en ce que l'espacement (z) entre les rangées d'entailles (15) correspond à peu près à la longueur (y) de la zone de recouvrement des entailles (14) de deux rangées d'entailles (15).

5. Vêtement selon l'une quelconque des revendications précédentes, caractérisé en ce que l'espacement (z) des rangées d'entailles (15) entre elles correspond à peu près à l'espacement (w) entre les entailles (14) d'une rangée d'entailles (15).

6. Vêtement selon l'une quelconque des revendications précédentes caractérisé en ce que l'espacement (z) entre les rangées d'entailles (14) est de l'ordre de 1 à 4 mm.

7. Vêtement selon l'une quelconque des revendications précédentes, caractérisé en ce que la longueur (x) d'une entaille (14) correspond à 2 à 4 fois la longueur de l'espacement z.

8. Vêtement selon l'une quelconque des revendications précédentes, caractérisé en ce que la longueur (x) d'une entaille (14) est de l'ordre de 3 z.
